# EUROPEAN PATENT APPLICATION

(11) **EP 4 663 141 A1**
(43) Date of publication of application: **17.12.2025**
(21) Application number: 25181962.9
(22) Date of filing: 11.06.2025
(51) Int. Cl.: A61B 18/12, A61B 18/14, A61B 34/30, A61B 34/37, A61B 18/00, A61B 17/00

(54) **ROBOTIC SURGICAL SYSTEM**

(30) Priority: 12.06.2024 JP 2024095292
(71) Applicant: Medicaroid Corporation, Kobe-shi, Hyogo 650-0047 (JP)
(72) Inventor: TAKAHASHI, Kaoru, Kobe-shi, Hyogo, 650-0047 (JP)
(74) Representative: Müller Hoffmann & Partner

(57) **Abstract**

In a robotic surgical system (500), a surgical instrument (201) includes an end effector (201a), a supply line (L1) that supplies electric energy to the end effector and a switch element (SW1) that turns on/off the supply line, an electrosurgical unit (210, 230) is configured to supply electrical energy to the surgical instrument, and a control device (300) is configured to transmit a signal to the switch element while an input is received by an input device (125, 126), wherein the switch element is configured to turn on the supply line while the signal from the control device is received.

## Description

### TECHNICAL FIELD

The disclosure may relate to a robotic surgical system.

### BACKGROUND

In a related art, there has been a robotic surgical system that includes a robotic arm to which an electrosurgical instrument is attached (for example, Patent Document 1: U.S. Patent No. 8,423,182). Patent Document 1 discloses a robotic surgical system comprising a robot arm, an electrosurgical instrument attached to the robot arm, and a plurality of electrosurgical units for supplying electric energy to the surgical instrument. In Patent Document 1, a robotic surgical system reads tool information of the surgical instrument from a memory of the surgical instrument attached to the robot arm, and receives device information from each of a plurality of electrosurgical units. The robotic surgical system automatically assigns an appropriate electrosurgical unit to the surgical instrument attached to the robot arm from among the plurality of electrosurgical units. This allows electrical energy to be supplied from the electrosurgical unit to the surgical instrument.

Patent Document 1: U.S. Patent No. 8,423,182

### SUMMARY

In the robot surgical system of Patent Document 1, while the robot surgical system receives device information from each of a plurality of electrosurgical units, when an electrosurgical unit not originally included in the robot surgical system is arranged, the robot surgical system cannot communicate with the electrosurgical unit and cannot obtain device information. That is, an electrosurgical unit that cannot communicate with the robotic surgical system cannot be used. Therefore, it is desirable to provide a robotic surgical system capable of supplying electrical energy to a surgical instrument using an electrosurgical unit in which the robotic surgical system cannot obtain device information.

An object of an embodiment of the present disclosure may be to provide a robotic surgical system capable of supplying electrical energy to a surgical instrument using an electrosurgical unit that cannot acquire device information by the robotic surgical system.

An aspect of the disclosure may be a robotic surgical system, that may include a surgical instrument that includes an end effector, a supply line that supplies an electric energy to the end effector and a switch element that turns on/off the supply line, a robot arm to which the surgical instrument is detachably attached, an electrosurgical unit that supplies the electric energy to the surgical instrument, an input device that receives an input instructing to perform a surgical procedure using the surgical instrument and one or more control devices that transmit a signal to the switch element while the input received by the input device, wherein the switch element turns on the supply line while the signal from the one or more control devices is received.

According to the aspect, as described above, the one or more control devices transmit the signal to the switch element while the input is received by the input device, and the switch element turns on the supply line while the signal from the one or more control devices is received. Thus, since the supply line is turned on by the switch element to which the signal is transmitted, the electric energy is supplied to the end effector through the supply line even if the robotic surgical system cannot receive the device information of the electrosurgical unit for supplying the electric energy. Thus, electrical energy can be supplied to the surgical instrument using the electrosurgical unit from which the robotic surgical system cannot obtain the device information.

According to the aspect, electrical energy can be supplied to a surgical instrument using an electrosurgical unit from which a robotic surgical system cannot obtain device information.

### BRIEF DESCRIPTION OF DRAWINGS

FIG. 1 is a diagram illustrating a configuration of a robotic surgical system according to an embodiment;
FIG. 2 is a diagram illustrating a display of a medical cart according to the one embodiment;
FIG. 3 is a diagram illustrating a configuration of the medical cart according to the one embodiment;
FIG. 4 is a diagram illustrating a configuration of a robot arm according to the one embodiment;
FIG. 5 is a diagram showing a forceps;
FIG. 6 is a diagram illustrating a configuration of an arm operation unit according to the one embodiment;
FIG. 7 is a diagram illustrating translational movement of the robot arm;
FIG. 8 is a diagram illustrating rotational movement of the robot arm;
FIG. 9 is a perspective diagram illustrating a foot pedal according to the one embodiment;
FIG. 10 is a control block diagram of a surgical robot according to the one embodiment;
FIG. 11 is a control block diagram of the robot arm according to the one embodiment;
FIG. 12 is a control block diagram of a positioner and the medical cart according to the one embodiment;
FIG. 13 is a diagram illustrating an endoscope;
FIG. 14 is a diagram illustrating a pivot-position setting tool;
FIG. 15 is a diagram illustrating configurations of an instrument and an electrosurgical unit according to a first embodiment;
FIG. 16 is a diagram for explaining a display state when the coagulation pedal or the cutting pedal is pressed on a monitor of a remote control apparatus and a display unit arranged on the medical cart;
FIG. 17 is a diagram illustrating configurations of an instrument and an electrosurgical unit according to a second embodiment;
FIG. 18 is a diagram illustrating configurations of an instrument and an electrosurgical unit according to a third embodiment;
FIG. 19 is a diagram illustrating configurations of an instrument and an electrosurgical unit according to a fourth embodiment;
FIG. 20 is a diagram illustrating configurations of an instrument and an electrosurgical unit according to a fifth embodiment; and
FIG. 21 is a diagram illustrating configurations of an instrument and an electrosurgical unit according to a sixth embodiment.

### DETAILED DESCRIPTION

Descriptions are provided hereinbelow for embodiments based on the drawings. In the respective drawings referenced herein, the same constituents are designated by the same reference numerals and duplicate explanation concerning the same constituents is omitted. All of the drawings are provided to illustrate the respective examples only.

### (Configuration of Robotic Surgical System)

The following description describes a configuration of a robotic surgical system 500 according to this embodiment. The robotic surgical system 500 includes a surgical robot 100, a remote operation apparatus 200, a third controller 300 and an image processing unit 400. The third controller 300 is an example of "one or more control devices".

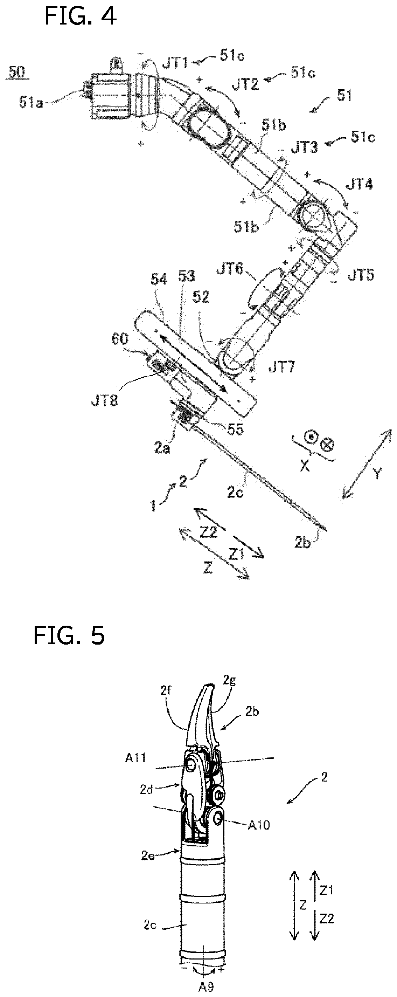

In this specification, a longitudinal direction of a surgical instrument 1 is defined as a Z direction as shown in FIG. 4. A fore-end side of the surgical instrument 1 is defined as a Z1 side, and a base-end side of the surgical instrument 1 is defined as a Z2 side. A direction orthogonal to the Z direction is defined as an X direction. A direction orthogonal to the Z direction and the X direction is defined as a Y direction.

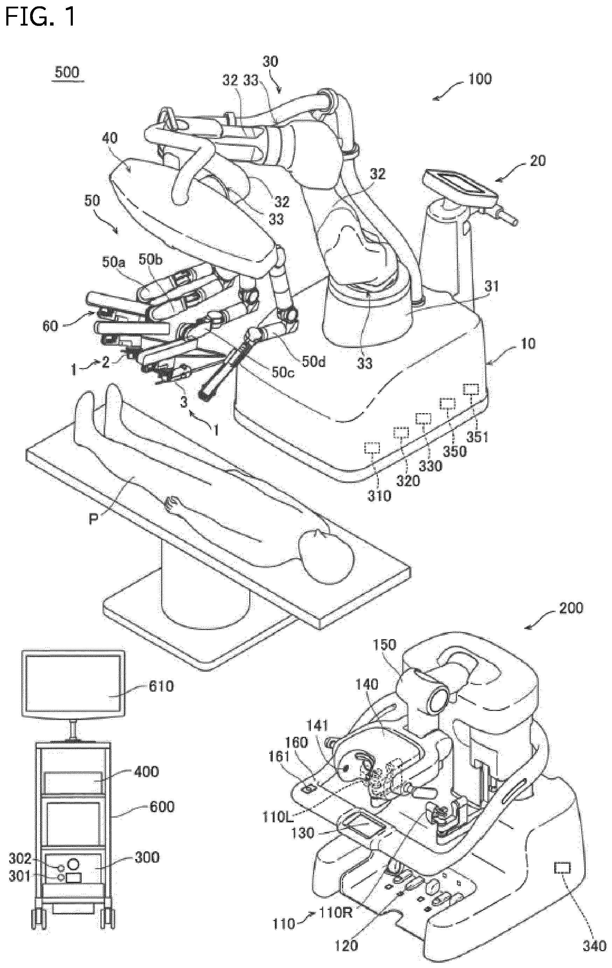

As shown in FIG. 1, the surgical robot 100 is arranged in an operating room. The remote operation apparatus 200 is located remote from the surgical robot 100. Also, the remote operation apparatus 200 is configured to receive operations as to the surgical instruments 1. Specifically, an operator, such as a doctor, can provide the remote operation apparatus 200 with an instruction to instruct a desired motion of the surgical robot 100. The remote operation apparatus 200 transmits the provided command to the surgical robot 100. The surgical robot 100 is configured to perform the motion in accordance with the command received. The surgical robot 100 is arranged in the operating room, which is a sterile field.

### (Configuration of Surgical Robot)

As shown in FIG. 1, the surgical robot 100 includes a medical cart 10, a cart positioner operation unit 20, a positioner 30, an arm base 40, a plurality of robot arms 50 and arm operation units 60.

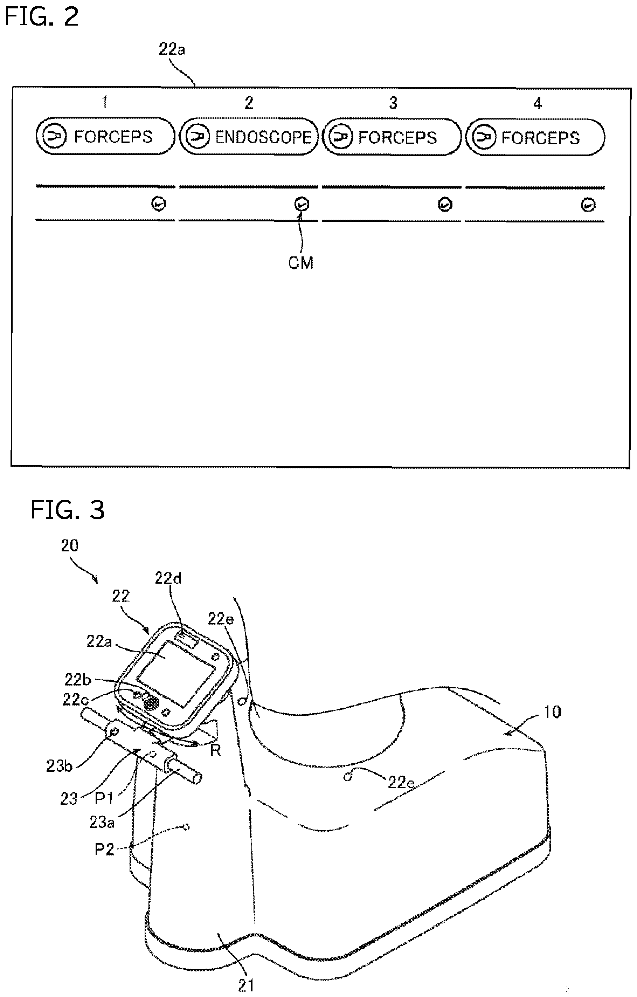

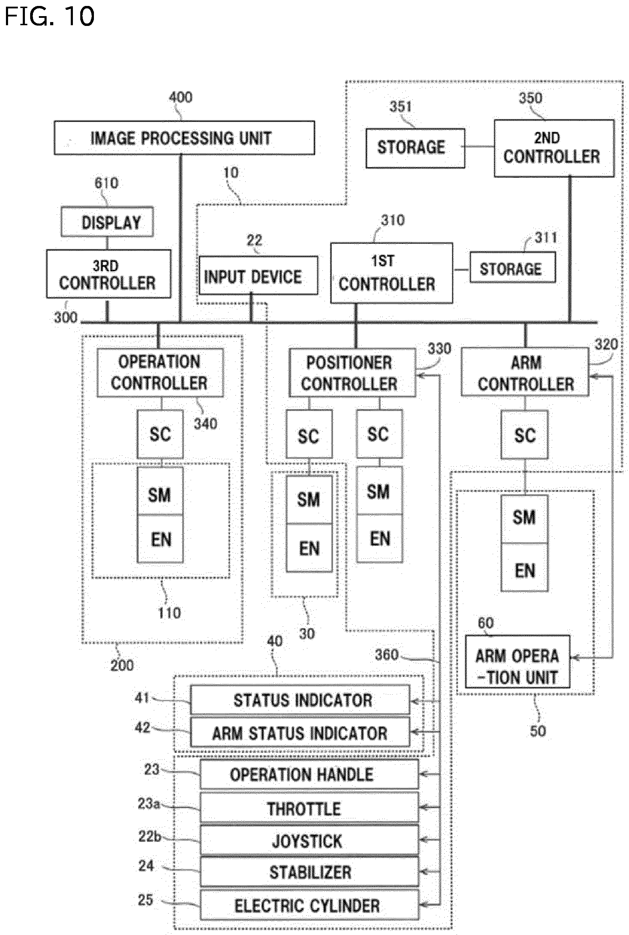

As shown in FIG. 3, the cart positioner operation unit 20 is arranged in a rear part of the medical cart 10 and supported by a cart positioner operation support 21, and the medical cart 10 or the positioner 30 can be moved in accordance with a manual operation of the cart positioner operation unit 20. The cart positioner operation unit 20 includes an input device 22 and an operation handle 23. The input device 22 is configured to accept operations to move or change orientations of the positioner 30, the arm base 40 and the plurality of robot arms 50 to prepare a surgical operation mainly before the operation is carried out. The medical cart 10 includes the operation handle 23, a stabilizer 24 and an electric cylinder 25 shown in FIG. 10. The stabilizer 24 includes an electric cylinder, and the movement of the medical cart 10 is suppressed by causing the wheels of the medical cart 10 to float from the ground by the stabilizer 24.

As shown in FIG. 3, the input device 22 of the medical cart 10 includes the display 22a, a joystick 22b, an enable switch 22c, an error reset button 22d and speakers 22e. For example, the display 22a is a liquid crystal panel. As shown in FIG. 2, the display 22a indicates numbers corresponding to the plurality of robot arms 50. Also, the display 22a indicates types of surgical instruments 1 attached to the plurality of robot arms 50. The display 22a indicates checkmarks CM representing that their pivot positions PP (discussed later) have been set.

As shown in FIG. 3, the joystick 22b is arranged in proximity to the display 22a of the input device 22 of the medical cart 10. When an operation mode displayed on the display 22a is selected, the positioner 30 can be three-dimensionally moved by operating the joystick 22b.

The enable switch 22c is arranged in proximity to the joystick 22b of the medical cart 10. The enable switch 22c is configured to enable or disable movement of the positioner 30. When the enable switch 22c is pressed so that movement of the positioner 30 is enabled, the positioner 30 can be moved in accordance with a manual operation of the joystick 22b.

The error reset button 22d is configured to reset an error of the robotic surgical system 500. An exemplary error is an error of abnormal deviation. The speakers 22e are a pair of speakers. The pair of speakers 22e are arranged at a position in the medical cart 10 in proximity to the positioner 30.

Also, the operation handle 23 is arranged in proximity to the display 22a of the medical cart 10. The operating handle 23 includes a throttle grip 23a that is configured to be gripped and twisted by an operator such as nurse, engineer, etc. to control movement of the medical cart 10. Specifically, the operation handle 23 is arranged under the input device 22. The medical cart 10 can move forward when the throttle grip 23a is twisted from a near side toward a far side. The medical cart 10 can move backward when the throttle grip 23a is twisted from the far side toward the near side. A speed of the medical cart 10 can be changed in accordance with a twisting amount of the throttle grip 23a. In addition, the operation handle 23 is configured to swing leftward and rightward as shown by an R direction, and to rotate the medical cart 10 depending on the swinging operation of the operation handle 23.

Also, the operation handle 23 of the medical cart 10 includes an enable switch 23b configured to enable or disable movement of the medical cart 10. When the enable switch 23b is pressed so that movement of the medical cart 10 is enabled, the medical cart 10 can be moved in accordance with a manual operation of the throttle grip 23a of the operating handle 23.

For example, as shown in FIG. 1, the positioner 30 is constructed of a 7-axis multi-joint robot. The positioner 30 is arranged on the medical cart 10. The positioner 30 is configured to adjust a position of the arm base 40. The positioner 30 can three-dimensionally move the position of the arm base 40.

The positioner 30 includes a base 31, and a plurality of links 32 coupled to the base 31. The links 32 are coupled to each other by joints 33.

The arm base 40 is attached to a free end of the positioner 30. The base ends of the plurality of robot arms 50 are attached to the arm base 40. The plurality of robot arms 50 are foldable into a storage posture. The arm base 40 and the plurality of robot arms 50 covered by sterile drapes when used. The robot arms 50 respectively support the surgical instrument 1.

A status indicator 41 and an arm status indicator 42 shown in FIG. 10 are provided in the arm base 40. The status indicator 41 is configured to indicate a status of robotic surgical system 500. The arm status indicator 42 is configured to indicate states of robot arms 50.

Two or more robot arms 50 are provided as a plurality of robot arms 50. Specifically, four robot arms 50a, 50b, 50c and 50d are provided. The robot arms 50a, 50b, 50c and 50d have a similar configuration to each other. The robot arms 50a and 50b are examples of "a second robot arm ". The robot arm 50d is an example of "a robot arm ".

As shown in FIG. 4, the robot arm 50 includes an arm portion 51, a first link portion 52, a second link portion 53, and a translation mechanism 54. The robot arm 50 includes, as joints 51c, rotation joints JT1, JT2, JT3, JT4, JT5, JT6 and JT7, and a linear motion joint JT8. The linear motion joint JT8 is a linear motion joint in which the translation mechanism 54 moves the second link portion 53 relative to the first link portion 52 in the Z direction. The arm portion 51 includes a base portion 51a, link portions 51b, and the rotation joints.

The arm portion 51 is constructed of a 7-axis multi-joint robot arm. The first link 52 is arranged in a distal end of arm portion 51. The arm operation unit 60 discussed later is attached to the second link 53. The translation mechanism 54 is arranged between the first link 52 and the second link 53. The second link 53 includes a holder 55 configured to hold the surgical instrument 1. The translation mechanism 54 is configured to translationally move the holder 55 to which the surgical instrument 1 is attached between a first position and a second position. The first position is a position of a Z2-direction side end of a moving range of the holder 55 moved by the translation mechanism 54 along the axis of the linear motion joint JT8. The second position is a position of a Z1-direction side end of the moving range of the holder 55 moved by the translation mechanism 54 along the axis of the joint JT8.

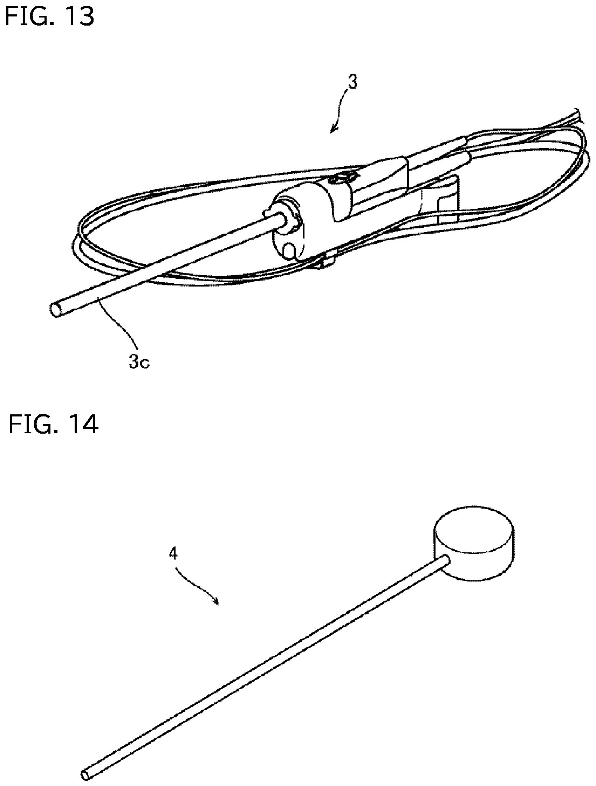

Surgical instruments 1 can be attached to the distal ends of the plurality of robot arms 50. The surgical instruments 1 include, for example, replaceable instruments, an endoscope 3 (see FIG. 13 ) configured to capture images of a part to be operated, a pivot-position setting tool 4 (see FIG. 14) to set a pivot position PP described below, etc. The instrument 2 includes a driven unit 2a, a forceps 2b and a shaft 2c.

As shown in FIG. 1, an endoscope 3 is attached to the free end of one, e.g., the robot arm 50c of the robot arms 50, and the instruments 2 are attached to the free ends of the others, e.g., the robot arms 50a, 50b and 50d. The endoscope 3 is attached to one of two robot arms 50b and 50c, which are located in a central part, of the four robot arms 50 arranged adjacent to each other.

### (Configuration of Instrument)

For example, as shown in FIG. 5, a forceps 2b is attached to the distal end of the instrument 2. Tools that include a joint and can be attached to the distal end of the instrument 2 can include scissors, a grasper, a needle holder, a micro dissector, a staple applier, a tucker, a vacuum cleaning tool, a snare wire, a clip applier, etc., other than the forceps 2b. Tools that do not include any joint and can be attached to the free end of the instrument 2 can include a cutting blade, a cautery probe, a cleaner, a catheters, a vacuum orifice, etc.

The forceps 2b includes a first support 2d and a second support 2e. The first support 2d is configured to rotatably support a base end side of jaws 2f and 2g about an axis A11. The second support 2e is rotatably configured to support a base-end side of the first support 2d about an axis A10. The shaft 2c can rotate about an axis A9. The jaws 2f and 2g can pivot about the axis A11 to open and close.

### (Configuration of Arm Operation Unit)

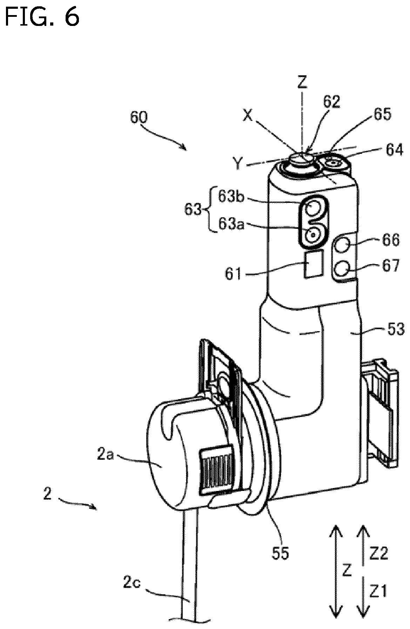

As shown in FIG. 6 , the arm operation unit 60 is mounted to the robot arm 50, and is configured to operate the robot arm 50. Specifically, the arm operation unit 60 is mounted to the second link 53.

The arm operation unit 60 include an enable switch 61, a joystick 62, linear switches 63, a mode switching button 64, a mode indicator 65, a pivot button 66, and an adjustment button 67.

The enable switch 61 is configured to enable or disable movement of the robot arm 50 by the joystick 62 and the linear switches 63 when pressed. Movement of the surgical instrument 1 by the robot arm 50 is enabled when the enable switch 61 is pressed while the arm operation unit 60 is grasped by an operator such as nurse, assistant, etc.

The joystick 62 is an operation tool configured to control movement of the surgical instrument 1 by the robot arm 50. The joystick 62 is an operation tool configured to control a moving direction and a moving speed of the robot arm 50. The robot arm 50 can be moved in accordance with to a tilting direction and a tilting angle of the joystick 62.

The linear switches 63 are a switch for moving the surgical instrument 1 in the Z direction, which is a longitudinal direction of the instrument 1. The linear switches 63 includes a linear switch 63a for moving the surgical instrument 1 in a direction in which the surgical instrument 1 is inserted into a patient P, and a linear switch 63b for moving the surgical instrument 1 in a direction in which the surgical instrument 1 is moved away from the patient P. The linear switch 63a and the linear switch 63b are constructed of a press-button switch.

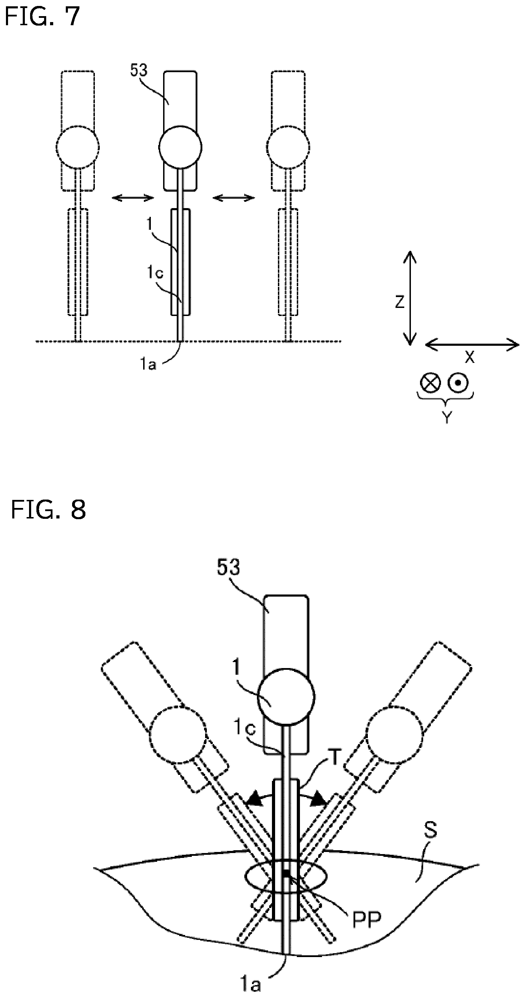

The mode switching button 64 is a press-button switch for switching between a translation mode in which the surgical instrument 1 is translationally moved, and a rotation mode in which the surgical instrument 1 is rotated. As shown in FIG. 7, in the translation mode in which the robot arm 50 is translationally moved, the robot arm 50 can be moved so that the distal end 1a of the surgical instrument 1 can be moved in an X-Y plane. As shown in FIG. 8, in the rotation mode in which the robot arm 50 is rotated, in a case in which any pivot position PP is not stored in the storage 351, the robot arm 50 can be moved so that the instrument 2 as the surgical instrument 1 rotates about a center of the forceps 2b on the axis A11, and in a case in which a pivot position PP is stored in the storage 351, the robot arm 50 can be moved so that the surgical instrument 1 rotates around the pivot position PP. In this case, the surgical instrument 1 is rotated with the shaft 1c of the surgical instrument 1 being inserted into a trocar T placed on a body surface S of the patient P. The mode switching button 64 is arranged on a surface on a Z-direction side of the arm operation unit 60.

The mode indicator 65 is configured to indicate which mode is selected. The mode indicator 65 is configured to light on to indicate the rotation mode, and to light off indicate the translation mode. The mode indicator 65 also serves as a pivot position indicator to indicate that the pivot position PP is set. The mode indicator 65 is arranged on the surface on the Z-direction side of the arm operation unit 60.

The pivot button 66 is a press-button switch configured to set the pivot position PP as a fulcrum point of movement of the surgical instrument 1 attached to the robot arm 50.

The adjustment button 67 is a button configured to optimize a position of the robot arm 50. After the pivot position PP is set with respect to the robot arm 50 to which the endoscope 3 is attached, when the adjustment button 67 is pressed positions of the other robot arms 50 and the arm base 40 is optimized. The adjustment button 67 is a button different from the enable switch 61.

### (Remote Operation Apparatus)

For example, as shown in FIG. 1, the remote operation apparatus 200 is arranged in an operating room or outside the operating room. The remote operation apparatus 200 includes operation units 110, foot pedals 120, a touch panel 130, a monitor 140, a support arm 150, a support bar 160, and an error reset button 161. The operation units 110 serves as a handle for operation that is configured to receive commands from an operator such as doctor.

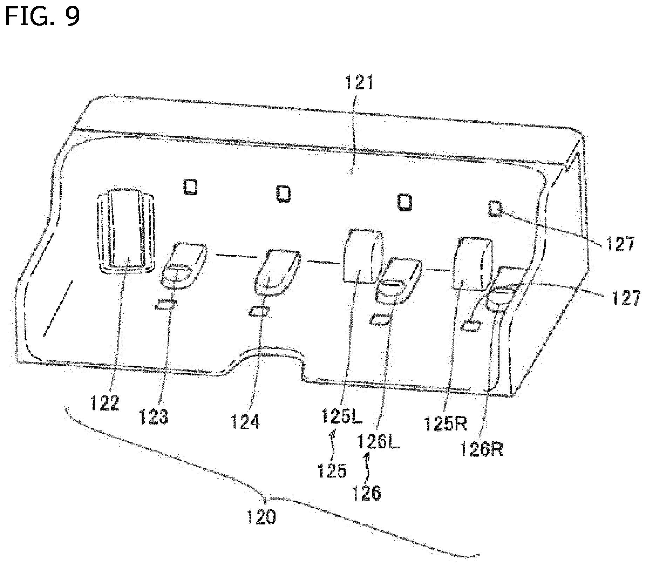

The operation units 110 are handle configured to manipulate the surgical instrument 1. Also, the operation units 110 are configured to receive operations as to the surgical instruments 1. The operation units 110 include an operation unit 110L that is arranged on a left side from viewpoint of an operator such as doctor and is configured to be manually operated by a left hand of the operator, and an operation unit 110R that is arranged on a right side from viewpoint of the operator and is configured to be manually operated by a right hand of the operator. The operation handle 110 L and the operation handle 110R operate the robot arm 50, respectively. For example, the endoscope 3 is attached to one of two robot arms 50 arranged in the center among the four robot arms 50. For example, as shown in FIG. 1, when the endoscope 3 is attached to the robot arm 50c, the operation handle 110 L operates the robot arm 50a or 50b, and the operation handle 110R operates the robot arm 50d. When the endoscope 3 is attached to the robot arm 50b, the operation handle 110 L operates the robot arm 50a, and the operation handle 110R operates the robot arm 50c or 50d. The robot arm 50b or 50c to which the endoscope 3 is attached is operated by the operation handle 110 L and the operation handle 110R while the camera pedal 124 shown in FIG. 9 is depressed. The operation handle 110R is an example of "a first operation device ", and the operation handle 110 L is an example of "a second operation device ".

The monitor 140 is a scope-type display device configured to display images captured by the endoscope 3. The monitor 140 includes an information producer 141. The information producer 141 is configured to produce an error sound. The support arm 150 supports the monitor 140, and can adjust a height of the monitor 140 to a height of eyes of the operator such as doctor. The touch panel 130 is arranged on the support bar 160. When a head of the operator is detected by a sensor arranged in proximity to the monitor 140, the surgical robot 100 can accept manual operations from the remote operation apparatus 200. The operator will manually operate the operation unit 110 and the foot pedals 120 while seeing of an affected area on the monitor 140. Commands can be provided to the remote operation apparatus 200 in accordance with these manual operations. Instructions provided to the remote operation apparatus 200 are transmitted to the surgical robot 100.

The error reset button 161 is arranged on the support bar 160. The error reset button 161 is configured to reset an error of the robotic surgical system 500. An exemplary error is an error of abnormal deviation.

As shown in FIG. 9, a plurality of foot pedals 120 configured to activate functions of the surgical instruments 1. The plurality of foot pedals 120 are provided in a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, the coagulation pedals 126 are configured to be operated by an operator's foot. Also, the incision pedals 125 include an incision pedal 125R corresponding to a right-side robot arm 50 and an incision pedal 125L corresponding to a left-side robot arm 50. Also, the coagulation pedals 126 include a coagulation pedal 126R corresponding to a right-side robot arm 50 and a coagulation pedal 126L corresponding to a left-side robot arm 50. Thus, since the operation handle 110R, the operation handle 110 L, the coagulation pedal 126 and the cutting pedal 125 are assembled in the remote operation device 200, the user can easily operate the operation handle 110R, the operation handle 110 L, the coagulation pedal 126 and the cutting pedal 125. Further, since the coagulating pedal 126R and the cutting pedal 125R are provided, the coagulating and cutting of the operation site can be performed by using the instrument 2 operated by the right hand. Similarly, since the coagulating pedal 126 L and the cutting pedal 125 L are provided, the instrument 2 operated with the left hand can be used to coagulate and cut the surgical site. The cutting pedal 125R is an example of "a first cutting pedal" and "an input device". The coagulation pedal 126R is an example of "a first coagulation pedal" and "an input device". The cutting pedal 125 L is an example of "a second cutting pedal" and "a second input device". The coagulation pedal 126 L is an example of "a second coagulation pedal" and "a second input device".

The switching pedal 122 is configured to switch between the robot arms 50 to be operated by the operation unit 110. The clutch pedal 123 is configured to activate a clutch function of temporally halting operation connection between the robot arm 50 and the operation unit 110. While the clutch pedal 123 is pressed by the operator, operations provided by the operation unit 110 is not transmitted to the robot arm 50. While the camera pedal 124 is pressed by the operator, the robot arm 50 that holds the endoscope 3 can be operated through the operation unit 110. While the cut pedal 125 or the coagulation pedal 126 is depressed by the operator, the instrument 2, which is an electrosurgical instrument, is activated and a surgical procedure (cutting or coagulation) is performed.

The foot detectors 127 are configured to detect the operator's foot that operates the foot pedals 120. Each of the foot detectors 127 is disposed corresponding to each of the foot pedals 120, and detects a foot that hovers above the corresponding foot pedal 120. The foot detectors 127 are arranged on the base 121.

As shown in FIG. 1, a cart 600 holds a third controller 300 and an image processing unit 400. The image processing unit 400 is configured to process images captured by the endoscope 3. A display 610 is arranged on the cart 600. The display 610 is configured to display images captured by the endoscope 3. An error reset button 301 and an information producer 302 are arranged on a third controller 300. The error reset button 301 is configured to reset an error of the robotic surgical system 500. An exemplary error is an error of abnormal deviation. The information producer 302 is configured to produce an error sound.

### (Configuration of Control System)

As shown in FIG. 10, the robotic surgical system 500 includes a first controller 310, an arm controller 320, a positioner controller 330, operation controllers 340, a second controller 350 and a third controller 300. In addition, the robotic surgical system 500 includes a storage 311 connected to the first controller 310, and a storage 351 connected to the second controller 350. As shown in FIG. 1, the second controller 350 and the storage 351 are arranged inside the medical cart 10, for example. The first controller 310 and the third controller 300 are examples of "one or more control devices".

The first controller 310 is accommodated in the medical cart 10, and configured to communicate with the arm controller 320 and the positioner controller 330 so that the robotic surgical system 500 is entirely controlled. Specifically, the first controller 310 is configured to control the arm controller 320, the positioner controller 330 and the operation controllers 340 by using the communications with them. The first controller 310 is connected to the arm controller 320, the positioner controller 330 and the operation controllers 340 through LAN, etc. The first controller 310 is arranged in the medical cart 10.

Each of the plurality of robot arms 50 includes the arm controller 320. In other words, a plurality of arm controllers 320 the number of which corresponds to the number of the plurality of robot arms 50 are included in the medical cart 10.

As shown in FIG. 10, the input device 22 is connected to the first controller 310 through LAN, etc. The status indicator 41, the arm status indicator 42, the operation handle 23, the throttle grip 23 a, the joystick 22 b, the stabilizer 24 and the electric cylinder 25 are connected to the positioner controller 330 through a wiring line 360 by means of a communication network that can share information with them by using serial communication. Although all of these status indicator 41, arm status indicator 42, etc. are connected to each other through one wiring line 360 in FIG. 10, wiring lines 360 are actually provided to each of the status indicator 41, the arm status indicator 42, the operation handle 23, the throttle grip 23 a, the joystick 22 b, the stabilizer 24 and the electric cylinder 25.

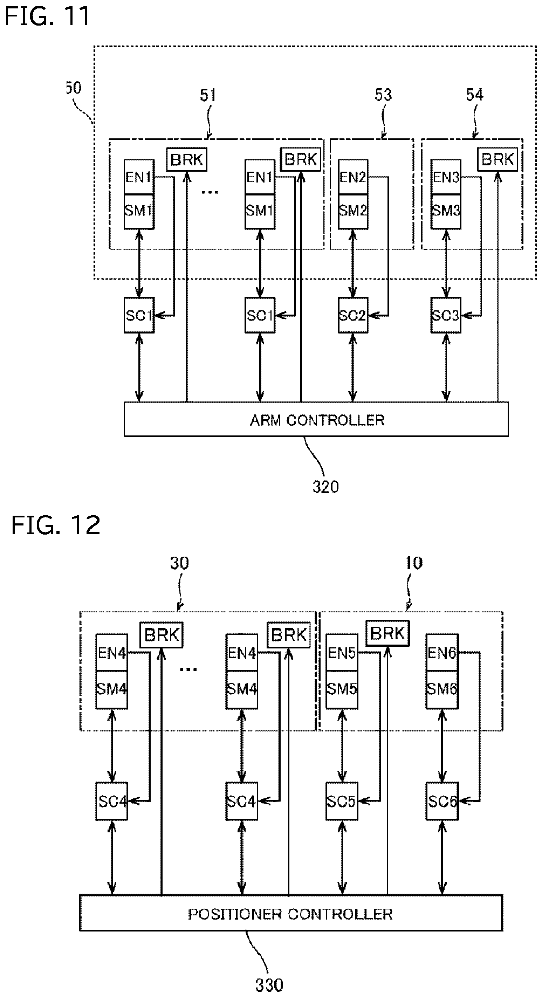

As shown in FIG. 11, each arm portion 51 includes a plurality of servomotors SM1, a plurality of encoders EN1 and a plurality of speed reducers corresponding to the Joints 51c. The encoder EN1 is configured to detect a rotation angle of the servomotor SM1. The speed reducer is configured to reduce a rotation of the servomotor SM1 whereby increasing its torque. A servo controller SC1 is configured to control the servomotor SM1, and is arranged in the medical cart 10 adjacent to the arm controller 320. Also, the encoder EN1 is configured to detect the rotation angle of the servomotor SM1, and is electrically connected to the servo controller SC1.

The second link 53 includes a servomotor SM2 configured to rotate a driven member arranged in a driven unit 2 a of the surgical instrument 1, an encoder EN2, and a speed reducer. The encoder EN2 is configured to detect a rotation angle of the servomotor SM2. The speed reducer is configured to reduce a rotation of the servomotor SM2 whereby increasing its torque. The medical cart 10 includes a servo controller SC2 configured to control the servomotor SM2 for driving the surgical instrument 1. The encoder EN2 for detecting the rotation angle of the servo motor SM2 is electrically connected to the servo control unit SC2. Note that a plurality of servomotors SM2, a plurality of encoders EN2 and a plurality of servo controllers SC2 are included.

The translation mechanism 54 includes a servomotor SM3 configured to translationally move the surgical instrument 1, an encoder EN3, and a speed reducer. The encoder EN3 is configured to detect a rotation angle of the servomotor SM3. The speed reducer is configured to reduce a rotation of the servomotor SM3 whereby increasing its torque. The medical cart 10 includes a servo controller SC3 configured to control the servomotor SM3 for translationally moving the surgical instrument 1. The encoder EN3 for detecting the rotation angle of the servo motor SM3 is electrically connected to the servo control unit SC3.

The first controller 310 is configured to generate instruction values that specify positions of the servomotor SM1, SM2 and SM3 in accordance with manual operation that is received by the operation unit 110 of the remote operation apparatus 200, and to drive the servomotor SM1, SM2 and SM3 in accordance with the instruction values. If any of differences between instruction values and positions of servomotor SM1, SM2 and SM3 detected by sensors becomes greater than an allowable range, the first controller 310 determines an error of abnormal deviation.

As shown in FIG. 12, the positioner 30 includes a plurality of servomotors SM4, a plurality of encoders EN4 and a plurality of speed reducers corresponding to a plurality of joints 33 of the positioner 30. Each encoder EN4 is configured to detect a rotation angle of the servomotor SM4. The speed reducer is configured to reduce a rotation of the servomotor SM4 whereby increasing its torque.

The medical cart 10 includes wheels including front wheels as driving wheels, and rear wheels configured to be steered by manually operating the handle 23. The rear wheels are arranged closer to the operating handle 23 with respect to the front wheels. The medical cart 10 includes a servomotor SM5 configured to drive the front wheels of the medical cart 10, an encoder EN5, speed reducers, and brakes BRK. The speed reducer is configured to reduce a rotation of the servomotor SM5 whereby increasing its torque. Also, the operation handle 23 of the medical cart 10 includes a potentiometer P1 shown in FIG. 3, and the servomotor SM5 of the front wheels can be driven in accordance with a rotation angle detected by the potentiometer P1 in response to a twisting amount of the throttle grip 23a. The rear wheels of the medical cart 10 have a twin-wheel type structure, and the rear wheels can be steered in accordance with a rightward/leftward turn of the operating handle 23. Also, the operation handle 23 of the medical cart 10 includes a potentiometer P2 shown in FIG. 3 on a turning shaft, and the rear wheel of medical cart 10 is provided with a servomotor SM6, an encoder EN6, and speed reducers. The speed reducer is configured to reduce a rotation of the servomotor SM6 whereby increasing its torque. The servomotor SM6 can be driven in accordance with a rotation angle detected by the potentiometer P2 in response to a rightward/leftward turning amount of the operation handle 23. In other words, power is assisted by the servomotor SM6 when the rear wheels are steered by turning the operation handle 23 rightward or leftward.

The medical cart 10 can be moved forward or rearward by driving the front wheels. Also, the medical cart 10 can be turned rightward or leftward by steering the rear wheels by turning the operating handle 23 of the medical cart 10.

As shown in FIG. 12, the medical cart 10 includes servo controllers SC4 configured to control the servomotors SM4 for moving the positioner 30. Also, the encoder EN4 is configured to detect the rotation angle of the servomotor SM4, and is electrically connected to the servo controller SC4. The medical cart 10 includes a servo controller SC5 configured to control the servomotor SM5 for driving the front wheels of the medical cart 10. The encoder EN5 for detecting the rotation angle of the servo motor SM5 is electrically connected to the servo controller SC5. The medical cart 10 includes a servo controller SC6 configured to control the servomotor SM6 for power assistance to steering of the rear wheels of the medical cart 10. The encoder EN6 for detecting the rotation angle of the servo motor SM6 is electrically connected to the servo control unit SC6.

As shown in FIGS. 11 and 12, the Joints 51c of the arm portion 51, and the joints 33 of the positioner 30 includes their brake BRK. Also, the front wheels of the medical cart 10, the arm base 40 and the translation mechanism 54 includes their brake BRK. The arm controller 320 is configured to one-directionally transmit control signals to the brakes BRK of the Joints 51c of the arm portion 51, and the translation mechanism 54. The control signals are configured to indicate on/off of the brakes BRK. The signals indicating on of the brakes BRK include a signal that instructs the brake BRK to keep activating. The control signals transmitted from the positioner controller 330 to the brakes BRK included in the joints 33 of the positioner 30 and the arm base 40 are configured similar to the control signals transmitted from the arm controller. On startup, all the brakes BRK of the arm base 40, the arm portion 51 and the translation mechanism 54 are turned off but the servomotors SM are driven to keep postures of the robot arm 50 and the arm base 40 against gravity. If an error occurs in the robotic surgical system 500, the brakes BRK included in the arm base 40, the arm 51 and the translation mechanism 54 are turned on. When the error in the robotic surgical system 500 is reset, the brakes BRK included in the arm base 40, the arm portion 51 and the translation mechanism 54 are turned off. When shutdown operation is performed in the robotic surgical system 500 is reset, the brakes BRK included in the arm base 40, the arm portion 51 and the translation mechanism 54 are turned on. The brakes BRK of the front wheels of the medical cart 10 are constantly turned on, and the brakes BRK are deactivated only when the enable switch 23b of the medical cart 10 is kept pressed. Also, the brakes BRK of the joints 33 of the positioner 30 are constantly turned on, and the brakes BRK are deactivated only when the enable switch 22 c of the medical cart 10 is kept pressed.

As shown in FIG. 10, the first controller 310 is configured to control the robot arm 50 in accordance with manual operations received by the arm operation unit 60. For example, the first controller 310 is configured to control the robot arm 50 in accordance with manual operations received by the joystick 62 of the arm control unit 60. Specifically, the arm controller 320 provides an input signal provided from the joystick 62 to the first controller 310. The first controller 310 generates position commands based on the received input signal and the rotation angles detected by the encoders EN1, and provides the position commands to the servo controllers SC1 via the arm controller 320. The servo controllers SC1 generate current commands based on the position commands provided from the arm controller 320 and the rotation angles detected by the encoders EN1, and provide the current commands to the servomotors SM1. Accordingly, the robot arm 50 is moved in accordance with an operation command provided to the joystick 62.

The first controller 310 controls the robot arm 50 based on an input signal from the linear switch 63 of the arm operation unit 60. Specifically, the arm controller 320 provides an input signal provided from the linear switch 63 to the first controller 310. The first controller 310 generates position commands based on the received input signal and the rotation angles detected by the encoder EN1 or EN3, and provides the position commands to the servo controller SC1 or SC3 via the arm controller 320. The servo controller SC1 or SC3 generate current commands based on the position commands provided from the arm controller 320 and the rotation angles detected by the encoder EN1 or EN3, and provide the current commands to the servomotor SM1 or SM3. Accordingly, the robot arm 50 is moved in accordance with an operation command provided to the linear switch 63.

The medical cart 10 includes the positioner controller 330. The positioner controller 330 is configured to control the positioner 30 and the medical cart 10. The positioner 30 includes a plurality of servomotors SM4, a plurality of encoders EN4 and a plurality of speed reducers corresponding to a plurality of joints 33 of the positioner 30. The medical cart 10 includes the servo controllers SC4 configured to control the servomotors SM4 of the positioner 30. The medical cart 10 includes servomotors SM5 and SM6 configured to drive the front wheels of the medical cart 10, the encoders EN5 and EN6, speed reducers, the servo controllers SC5 and SC6, and brakes BRK.

The operation controllers 340 are provided in a main body of the remote operation apparatus 200. The operation controllers 340 are configured to control the operating units 110. The operation controllers 340 are associated with both the left-hand side operation unit 110L and the right-hand side operation unit 110R. The operation unit 110 includes servomotors SM, encoders EN and speed reducers corresponding to the plurality of joints of the operation unit 110. The servo controllers SC configured to control the servomotors SM of the operation unit 110 is provided in the main body of the remote operation apparatus 200 adjacent to the operation controllers 340.

As shown in FIG. 10, the third controller 300 and the image processing unit 400 are connected to the first controller 310 through LAN. The display 610 is connected to the third controller 300.

### (Composition of Instruments and Electrosurgical Units)

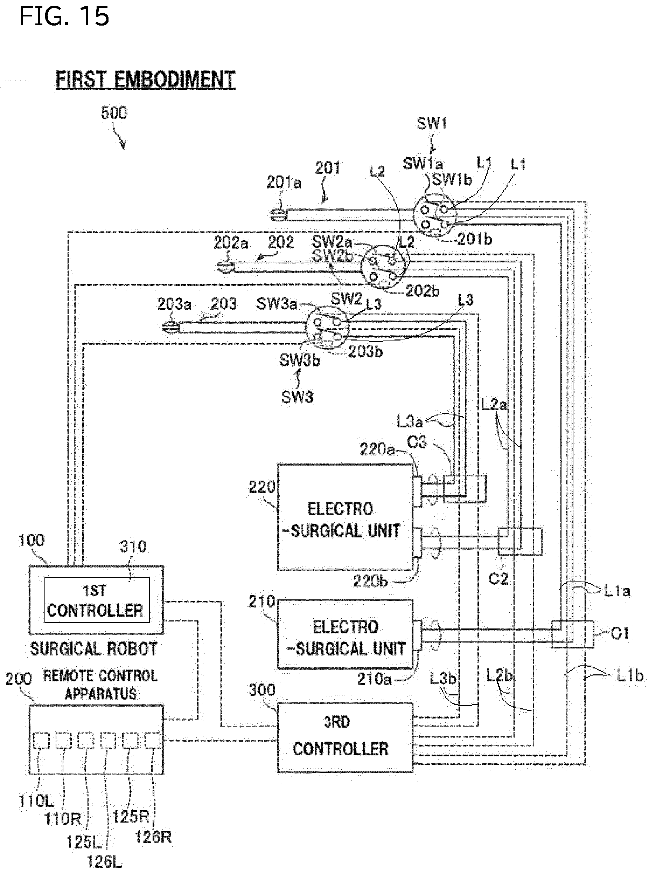

As described above, the instrument 2 is detachably attached to the robot arms 50a, 50b and 50d. As shown in FIG. 15, a plurality of kinds of instruments 2 to be attached to the robot arm 50 are prepared. For example, instrument 202 is a monopolar electrosurgical instrument with tissue dissection, cutting, and coagulation (sealing) functions, and instrument 203 is a bipolar electrosurgical instrument with dissection and coagulation functions. The instrument 201 is a different type of instrument from the instrument 202 and the instrument 203, and is a bipolar electrosurgical instrument having a sealing & cutting function in addition to a dissection function and a coagulation function. In the first embodiment, the instrument 201 includes an end effector 201a, a memory 201b storing tool information of the instrument 201, and a switch element SW1. The instrument 202 includes an end effector 202a, a memory 202b storing tool information of the instrument 202, and a switch element SW2. The instrument 203 includes an end effector 203a, a memory 203b storing tool information of the instrument 203, and a switch element SW3. End effector 201a, end effector 202a and end effector 203a are, as described above, e.g., forceps. The tool information of the instrument 201 includes the type of the instrument 201, information of the electrosurgical unit 210 that supplies electrical energy to the instrument 201, and the like. The tool information of the instrument 202 and the tool information of the instrument 203 also include similar information. Memory 201b, memory 202b and memory 203b are, for example, non-volatile memories. The switch element SW1, the switch element SW2, and the switch element SW3 are, for example, semiconductor switches. The instrument 201, the end effector 201a, and the memory 201b are examples of "a surgical instrument", "an end effector", and "a memory", respectively. The switch element SW1 is an example of "a switch element". The instrument 202, the end effector 202a, the memory 202b, and the switch element SW2 are examples of "a second surgical instrument", "a second end effector", "a second memory", and "a second switch element", respectively. The instrument 203, the end effector 203a, the memory 203b, and the switch element SW3 are examples of "a second surgical instrument", "a second end effector", "a second memory", and "a second switch element", respectively.

The electrosurgical unit 210 supplies electrical energy to the instrument 201. The electrical energy supplied by the electrosurgical unit 210 includes, for example, coagulating electrical energy for coagulating the surgical site and cutting electrical energy for cutting the surgical site. The electrosurgical unit 220 supplies electrical energy to each of the instruments 202 and 203. The electrical energy supplied by the electrosurgical unit 220 includes, for example, coagulating electrical energy for coagulating the surgical site and cutting electrical energy for cutting the surgical site. The first controller 310, the second controller 350 and the third controller 300 of the robotic surgical system 500 cannot receive the device information of the electrosurgical unit 210 and the electrosurgical unit 220 from the electrosurgical unit 210 and the electrosurgical unit 220.

In the first embodiment, the instrument 201 includes an electric supply line L1 that supplies electrical energy from the electrosurgical unit 210 to the end effector 201a, and the switch element SW1 is configured to turn on and off the electric supply line L1. That is, when the switch element SW1 is turned on, the electrical energy supplied from the electrosurgical unit 210 to the instrument 201 is supplied to the end effector 201a. When the switch element SW1 is turned off, the supply of the electrical energy from the electrosurgical unit 210 to the end effector 201a is stopped. The supply line L1 is an example of "a supply line".

In the first embodiment, the supply line L1 includes a coagulation supply line L1 for supplying coagulation electrical energy to the end effector 201a, and a cutting supply line L1 for supplying cutting electrical energy to the end effector 201a. The switch element SW1 includes a switch element SW1a arranged in the solidification supply line L1 and a switch element SW1b arranged in the cutting supply line L1. That is, the solidification supply line L1 and the cutting supply line L1 are provided separately, and are turned on/off by the switch elements SW1a and SW1b, respectively.

Also, in the first embodiment, the switch element SW2 is configured to turn on/off the supply line L2 for supplying electrical energy from the electrosurgical unit 220 to the end effector 202a. That is, when the switch element SW2 is turned on, the electrical energy supplied from the electrosurgical unit 220 to the instrument 202 is supplied to the end effector 202a. When the switch element SW2 is turned off, the supply of electrical energy from the electrosurgical unit 220 to the end effector 202a is stopped. Similarly, switch element SW3 is configured to turn on and off supply line L3 that supplies electrical energy from electrosurgical unit 220 to end effector 203a. When the switch element SW3 is turned on, the electrical energy supplied from the electrosurgical unit 220 to the instrument 203 is supplied to the end effector 203a. When the switch element SW3 is turned off, the supply of electrical energy from the electrosurgical unit 220 to the end effector 203a is stopped.

The supply line L2 includes a coagulation supply line L2 for supplying coagulation electrical energy to the end effector 202a and a cutting supply line L2 for supplying cutting electrical energy to the end effector 202a. The switch element SW2 includes a switch element SW2a arranged in the solidification supply line L2 and a switch element SW2b arranged in the cutting supply line L2. Similarly, supply line L3 includes a coagulation supply line L3 for supplying coagulation electrical energy to end effector 203a and a cutting supply line L3 for supplying cutting electrical energy to end effector 203a. The switch element SW3 includes a switch element SW3a arranged in the solidification supply line L3 and a switch element SW3b arranged in the cutting supply line L3. The supply lines L2 and L3 are examples of "a second supply line".

Further, in the first embodiment, the instrument 201 and the electrosurgical unit 210 are connected by an electric line L1a, and the instrument 201 and the third controller 300 are connected by an electric line L1b. Thus, electrical energy can be supplied from the electrosurgical unit 210 to the instrument 201 via the electric line L1a. Moreover, a signal for turning on/off the switch element SW1 of the instrument 201 can be transmitted from the third controller 300 via the electric line L1b. Similarly, the instrument 202 and the electrosurgical unit 220 are connected by an electric line L2a, and the instrument 202 and the third controller 300 are connected by an electric line L2b. The instrument 203 and the electrosurgical unit 220 are connected to each other by an electric line L3a, and the instrument 203 and the third controller 300 are connected to each other by an electric line L3b. The electric line L1a and the electric line L2a are examples of "a first electric line" and "a second electric line", respectively.

In the first embodiment, the electric line L1a includes a relay connector C1 between the instrument 201 and the electrosurgical unit 210, and the electric line L1b connects the instrument 201 and the third controller 300 via the relay connector C1. That is, the electric line between the electrosurgical unit 210 and the relay connector C1 and the electric line between the third controller 300 and the relay connector C1 are branched, while the electric line between the instrument 201 and the relay connector C1 is unified. Thus, it is possible to suppress the loose electric line between the relay connector C1 and the instrument 201. Similarly, the electric line L2a includes a relay connector C2 between the instrument 202 and the electrosurgical unit 220, and the electric line L2b connects the instrument 202 and the third controller 300 via the relay connector C2. The electric line L3a has a relay connector C3 between the instrument 203 and the electrosurgical unit 220, and the electric line L3b connects the instrument 203 and the third controller 300 via the relay connector C3.

The electrosurgical unit 210 also includes a connector 210a, and an electric line L1a is connected to the connector 210a. The electrosurgical unit 220 includes a connector 220a and a connector 220b, with electric lines L2a and L3a connected to the connectors 220a and 220b, respectively.

A description will now be given of a case where the instrument 202 is mounted on the robot arm 50 (For example, robot arm 50d) operated by the operation handle 110R, and the instrument 203 is mounted on the robot arm 50 (For example, robotic arm 50a or 50b) operated by the operation handle 110 L. When the instrument 202 is attached to the robot arm 50 operated by the operation handle 110R, the first controller 310 acquires the tool information of the instrument 202 from the memory 202b. The first controller 310 determines whether or not to assign the coagulation pedal 126R and the cutting pedal 125R to the instrument 202 based on the acquired tool information. If the instrument 202 is an electrosurgical instrument, the first controller 310 assigns the coagulation pedal 126R and the cutting pedal 125R to the instrument 202 mounted on the robotic arm 50 operated by the operating handle 110R. When the instrument 203 is attached to the robot arm 50 operated by the operation handle 110 L, the first controller 310 acquires tool information of the instrument 203 from the memory 203b. The first controller 310 determines whether or not to assign the coagulation pedal 126 L and the cutting pedal 125 L to the instrument 203 based on the acquired tool information. If the instrument 203 is an electrosurgical instrument, the first controller 310 assigns the coagulation pedal 126 L and the cutting pedal 125 L to the instrument 203 mounted on the robotic arm 50 operated by the operating handle 110 L. Thus, the instrument 202 operated by the right hand by the operation handle 110R, the coagulation pedal 126R and the cutting pedal 125R can be operated, and the instrument 203 operated by the left hand by the operation handle 110 L, the coagulation pedal 126 L and the cutting pedal 125 L can be operated.

In the first embodiment, the third controller 300 is configured to transmit an ON signal to the switch element SW2a or the switch element SW2b while the coagulation pedal 126R or the cutting pedal 125R is operated. The switch element SW2a or the switch element SW2b is configured to turn on the coagulation supply line L2 or the cutting supply line L2 while receiving the ON signal from the third controller 300. Thus, electrical energy for coagulation or electrical energy for cutting, which has been supplied from the electrosurgical unit 220 to the instrument 202 via the electric line L2a, is supplied to the end effector 202a. Further, the third controller 300 stops the transmission of the ON signal to the switch element SW2a or the switch element SW2b in response to the release of the operation of the coagulation pedal 126R or the cutting pedal 125R. Similarly, the third controller 300 is configured to transmit an ON signal to the switch element SW3a or the switch element SW3b while the coagulation pedal 126 L or the cutting pedal 125 L is operated. The switch element SW3a or the switch element SW3b is configured to turn on the coagulation supply line L3 or the cutting supply line L3 while receiving the ON signal from the third controller 300. Thus, electrical energy for coagulation or electrical energy for cutting, which has been supplied from the electrosurgical unit 220 to the instrument 203 via the electric line L3a, is supplied to the end effector 203a. In the first embodiment, the coagulation pedal 126R and the cutting pedal 125R are assigned to the instrument 202 based on the information of the robot arm 50 to which the instrument 202 is attached and the tool information. As a result, since the supply line L2 is turned on by the switch element SW2 while the coagulation pedal 126R or the cutting pedal 125R is operated, the electric energy is supplied from the electrosurgical unit 220 to the end effector 202a via the supply line L2 even if the robotic surgical system 500 does not exchange the device information of the electrosurgical unit 220. Thus, the electrosurgical unit 220, in which the robotic surgical system 500 cannot receive the device information by communication, can be used to provide electrical energy to the instrument 202. Similarly, the robotic surgical system 500 may provide electrical energy to the instrument 203 using the electrosurgical unit 220 that is unable to receive the device information by communication.

Next, a case where the instrument 202 is replaced and the instrument 201 is attached to the robot arm 50 will be described.

In the first embodiment, when the instrument 201 is attached to the robot arm 50, the first controller 310 acquires the tool information of the instrument 201 from the memory 201b. Then, the first controller 310 determines whether or not to assign the coagulation pedal 126R and the cutting pedal 125R to the instrument 201 based on the acquired tool information. If the instrument 201 is an electrosurgical instrument, the first controller 310 assigns the coagulation pedal 126R and the cutting pedal 125R to the instrument 201 mounted on the robotic arm 50 operated by the operation handle 110R. The third controller 300 is configured to transmit an ON signal to the switch element SW1a or the switch element SW1b while the coagulation pedal 126R or the cutting pedal 125R is operated. The switch element SW1 is configured to turn on the supply line L1 while receiving the ON signal from the third controller 300. The switch element SW1a or the switch element SW1b is configured to turn on the coagulation supply line L1 or the cutting supply line L1 while receiving the ON signal from the third controller 300. Thereby, even if the instrument 202 is replaced with the instrument 201, since the supply line L1 is turned on by the switch element SW1 while the coagulation pedal 126R or the cutting pedal 125R is operated, electrical energy is supplied to the end effector 201a from the electrosurgical unit 210 via the supply line L1 even if the robotic surgical system 500 cannot obtain device information from the electrosurgical unit 210. Thus, even when the electrosurgical unit 210 is arranged, in which the robotic surgical system 500 cannot obtain device information by communication, the instrument 201 can be supplied with electrical energy from the electrosurgical unit 210.

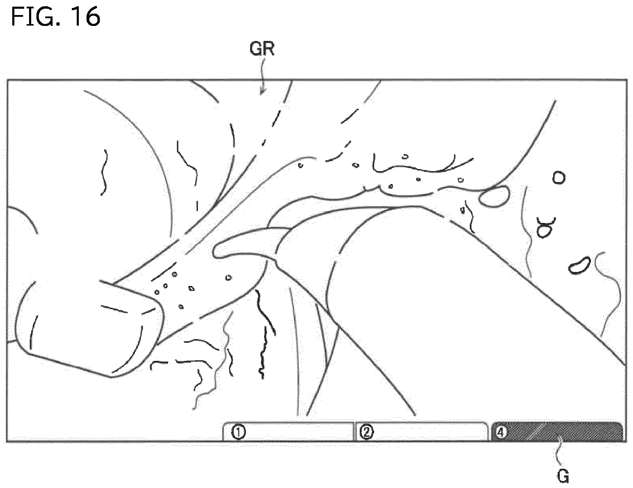

Further, as shown in FIG. 16, the third controller 300 generates a graphical user interface and displays the graphical user interface on the monitor 140 of the remote operation device 200 and the display unit 610 arranged on the cart 600, overlaying the image GR captured by the endoscope 3. The graphical user interface includes display areas corresponding to robot arms to which the instrument is attached to display the state of the coagulation pedal 126 and the disconnection pedal 125 respectively. For example, when the coagulation pedal 126R is depressed to perform coagulation treatment by the instrument attached to the robot arm 50d operated by the operation handle 110R, the color of the display area G of the numeral 4 corresponding to the robot arm 50d changes to the first color. In FIG. 16, the first color is represented by hatching. When the cutting pedal 125R is depressed to perform the cutting treatment by the instrument attached to the robot arm 50d, the color of the display area G of the numeral 4 changes to a second color different from the first color.

### (Second Embodiment)

The configuration of the robotic surgical system 500a according to a second embodiment will be described.

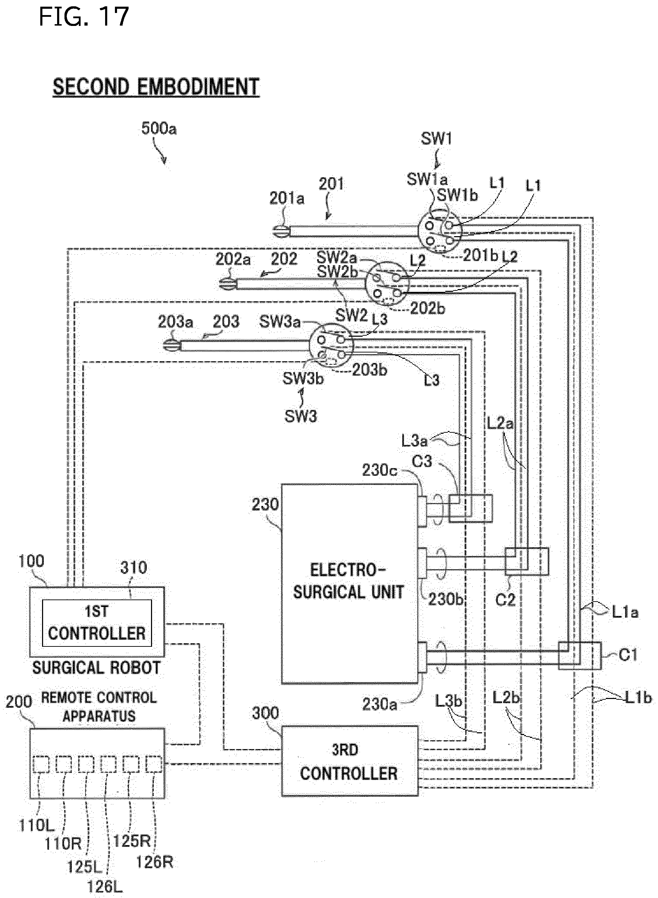

As shown in FIG. 17, the robotic surgical system 500a differs from the first embodiment in that one electrosurgical unit 230 is arranged. The electrosurgical unit 230 includes a connector 230a, a connector 230b, and a connector 230c, with an electric line L1a, an electric line L2a, and an electric line L3a connected to the connector 230a, the connector 230b, and the connector 230c, respectively. The electrosurgical unit 230 is configured to supply electrical energy to the instruments 201, 202 and 203. Thus, since the electrosurgical unit 230 is commonly provided for the instrument 201, the instrument 202, and the instrument 203, the configuration of the robotic surgical system 500a becomes simpler than in the case where the electrosurgical units are individually provided for the instrument 201, the instrument 202, and the instrument 203. The other configuration of the second embodiment is the same as that of the first embodiment.

### (Third Embodiment)

The configuration of the robotic surgical system 500b according to a third embodiment will be described.

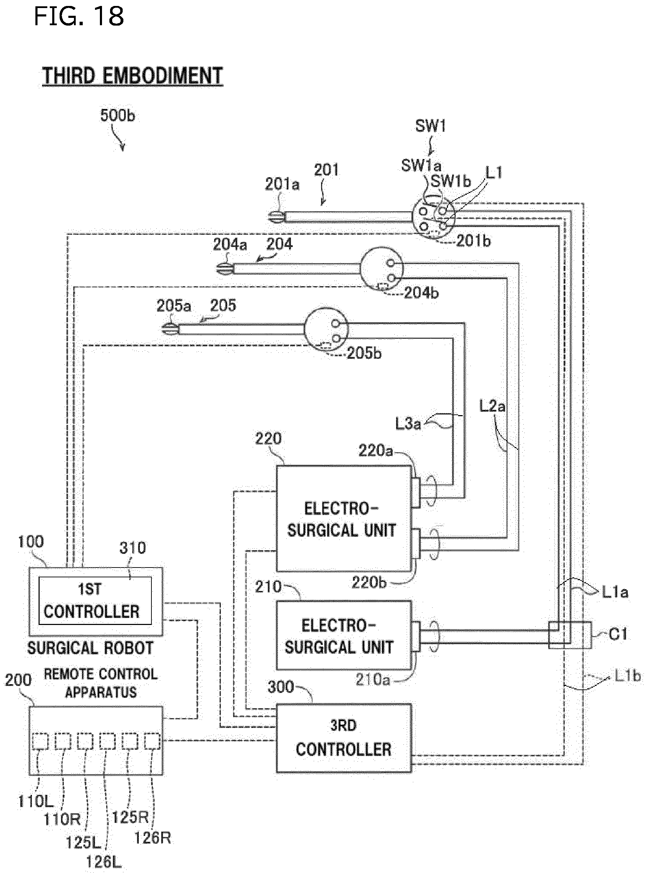

As shown in FIG. 18, the robotic surgical system 500b differs from the first and second embodiments in that the instruments 204 and 205 are not provided with switch elements. The instrument 204 includes a memory 204b storing tool information of the end effector 204a and the instrument 204. The instrument 205 also includes a memory 205b storing tool information of the end effector 205a and the instrument 205. The first controller 310, the second controller 350 and the third controller 300 of the robotic surgical system 500b cannot receive device information of the electrosurgical unit 220 from the electrosurgical unit 220. Specifically, the third controller 300 and the electrosurgical unit 220 are connected, and the third controller 300 can transmit an ON signal to the electrosurgical unit 220, but the third controller 300 cannot receive the device information of the electrosurgical unit 220. The electrosurgical unit 220 supplies electric energy to the instrument 204 and the instrument 205 when the ON signal is transmitted from the third controller 300. Thus, since the third controller 300 transmits the ON signal to the electrosurgical unit 220, electrical energy can be supplied from the electrosurgical unit 220 to the end effector 204a of the instrument 204 and the end effector 205a of the instrument 205. The instrument 204 is an example of "a second electrosurgical instrument". The end effector 204a and the memory 204b are examples of "a second end effector" and "a second memory", respectively. The instrument 205 is an example of "a third electrosurgical instrument". The end effector 205a and the memory 205b are examples of "a third end effector" and "a third memory", respectively.

When the instrument 204 is attached to the robot arm 50 operated by the operation handle 110R, the first controller 310 acquires the tool information of the instrument 204 from the memory 204b. The first controller 310 determines whether or not to assign the coagulation pedal 126R and the cutting pedal 125R to the instrument 204 based on the acquired tool information. If the instrument 204 is an electrosurgical instrument, the first controller 310 assigns the coagulation pedal 126R and the cutting pedal 125R to the instrument 204 mounted on the robotic arm 50 operated by the operating handle 110R. When the instrument 205 is attached to the robot arm 50 operated by the operation handle 110 L, the first controller 310 acquires tool information of the instrument 205 from the memory 205b. The first controller 310 determines whether or not to assign the coagulation pedal 126 L and the cutting pedal 125 L to the instrument 205 based on the acquired tool information. If the instrument 205 is an electrosurgical instrument, the first controller 310 assigns the coagulation pedal 126 L and the cutting pedal 125 L to the instrument 205 mounted on the robotic arm 50 operated by the operating handle 110 L.

In the third embodiment, the third controller 300 is configured to transmit an ON signal for coagulation or for cutting to the electrosurgical unit 220 while the coagulation pedal 126R or the cutting pedal 125R is operated. While receiving the ON signal, the electrosurgical unit 220 supplies the instrument 204 with coagulation or cutting electrical energy via an electric line L2a, and the supplied coagulation or cutting electrical energy is supplied to the end effector 204a via an electro supply line (not shown) in the instrument 204. The third controller 300 is configured to stop the transmission of the ON signal to the electrosurgical unit 220 in response to the release of the operation of the coagulation pedal 126R or the cutting pedal 125R. When the reception of the ON signal is stopped, the electrosurgical unit 220 stops the supply of the coagulation or cutting electrical energy to the instrument 204. Similarly, the third controller 300 is configured to transmit an ON signal to the electrosurgical unit 220 while the coagulation pedal 126 L or the cutting pedal 125 L is operated. While receiving the ON signal, the electrosurgical unit 220 supplies the instrument 205 with coagulation or cutting electrical energy via an electric line L3a, and the supplied coagulation or cutting electrical energy is supplied to the end effector 205a via a supply line (not shown) of the instrument 205. The third controller 300 is configured to stop the transmission of the ON signal to the electrosurgical unit 220 in response to the release of the operation of the coagulation pedal 126 L or the cutting pedal 125 L. When the reception of the ON signal is stopped, the electrosurgical unit 220 stops the supply of the coagulation or cutting electrical energy to the instrument 205. Thus, electrical energy is supplied from the electrosurgical unit 220 to the end effectors 204a and 205a via the supply lines L2a and L3a without the robotic surgical system 500b receiving the device information of the electrosurgical unit 220. Therefore, even if the electrosurgical unit 220 from which the robotic surgical system 500b cannot obtain device information is arranged, the instruments 204 and 205 can be supplied with electrical energy from the electrosurgical unit 220.

Next, a case where the instrument 201 is attached to the robot arm 50 in place of the instrument 204 will be described. In the third embodiment, when the instrument 201 is attached to the robot arm 50, the first controller 310 acquires the tool information of the instrument 201 from the memory 201b. Then, the first controller 310 determines whether or not to assign the coagulation pedal 126R and the cutting pedal 125R to the instrument 201 based on the acquired tool information. If the instrument 201 is an electrosurgical instrument, the first controller 310 assigns the coagulation pedal 126R and the cutting pedal 125R to the instrument 201 mounted on the robotic arm 50 operated by the operation handle 110R. The third controller 300 is configured to transmit an ON signal to the switch element SW1a or the switch element SW1b while the coagulation pedal 126R or the cutting pedal 125R is operated. The switch element SW1a or the switch element SW1b is configured to turn on the coagulation supply line L1 or the cutting supply line L1 while receiving the ON signal from the third controller 300. Thus, even when the instrument 204 is replaced with the instrument 201, since the supply line L1 is turned on by the switch element SW1 while the coagulation pedal 126R or the cutting pedal 125R is operated, the electrical energy that has been supplied from the electrosurgical unit 210 to the instrument 201 via the electric line L1a, is supplied to the end effector 201a via the supply line L1 even if the robotic surgical system 500b does not receive the device information of the electrosurgical unit 210. Thus, even if the electrosurgical unit 210 in which the robotic surgical system 500b cannot receive device information is disposed, the instrument 201 can be supplied with electrical energy from the electrosurgical unit 210.

The third embodiment differs from the first and second embodiments in that the wiring L2b and the wiring L3b are not provided. Therefore, the relay connector C2 and the relay connector C3 are not provided in the third embodiment.

### (Fourth Embodiment)

The configuration of the robotic surgical system 500c according to a fourth embodiment will be described.

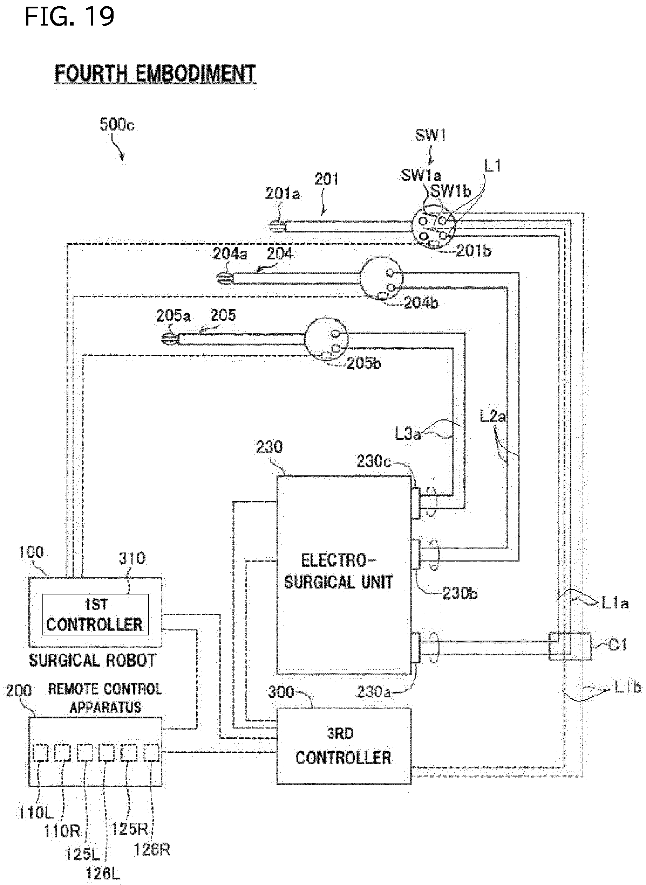

As shown in FIG. 19, the robotic surgical system 500c differs from the third embodiment in that one electrosurgical unit 230 is arranged. The electrosurgical unit 230 includes a connector 230a, a connector 230b, and a connector 230c, with an electric line L1a, an electric line L2a, and an electric line L3a connected to the connector 230a, the connector 230b, and the connector 230c, respectively. The first controller 310, the second controller 350, and the third controller 300 of the robotic surgical system 500c cannot receive the device information of the electrosurgical unit 230 from the electrosurgical unit 230. Specifically, the third controller 300 and the electrosurgical unit 230 are connected, and the third controller 300 can transmit an ON signal to the electrosurgical unit 230, but the third controller 300 cannot receive the device information of the electrosurgical unit 230.

When the instrument 204 is attached to the robot arm 50 operated by the operation handle 110R, the first controller 310 acquires the tool information of the instrument 204 from the memory 204b. The first controller 310 determines whether or not to assign the coagulation pedal 126R and the cutting pedal 125R to the instrument 204 based on the acquired tool information. If the instrument 204 is an electrosurgical instrument, the first controller 310 assigns the coagulation pedal 126R and the cutting pedal 125R to the instrument 204 mounted on the robotic arm 50 operated by the operation handle 110R. When the instrument 205 is attached to the robot arm 50 operated by the operation handle 110 L, the first controller 310 acquires tool information of the instrument 205 from the memory 205b. The first controller 310 determines whether or not to assign the coagulation pedal 126 L and the cutting pedal 125 L to the instrument 205 based on the acquired tool information. If the instrument 205 is an electrosurgical instrument, the first controller 310 assigns the coagulation pedal 126 L and the cutting pedal 125 L to the instrument 205 mounted on the robotic arm 50 operated by the operation handle 110 L.

In the fourth embodiment, the third controller 300 is configured to transmit an ON signal to the electrosurgical unit 230 while the coagulation pedal 126R or the cutting pedal 125R is operated. While receiving the ON signal, the electrosurgical unit 230 supplies electrical energy for coagulation or electrical energy for cutting to the end effector 204a of the instrument 204 via the electric line L2a. The third controller 300 is configured to stop the transmission of the ON signal to the electrosurgical unit 230 in response to the release of the operation of the coagulation pedal 126R or the cutting pedal 125R. When the reception of the ON signal is stopped, the electrosurgical unit 230 stops the supply of electric energy for coagulation or electric energy for cutting to the instrument 204. Similarly, the third controller 300 is configured to transmit an ON signal to the electrosurgical unit 230 while the coagulation pedal 126 L or the cutting pedal 125 L is operated. While receiving the ON signal, the electrosurgical unit 230 supplies electrical energy for coagulation or electrical energy for cutting to the end effector 205a of the instrument 205 via the electric line L3a. The third controller 300 is configured to stop the transmission of an ON signal to the electrosurgical unit 230 in response to the release of the operation of the coagulation pedal 126 L or the cutting pedal 125 L. When the reception of the ON signal is stopped, the electrosurgical unit 220 stops the supply of electric energy for coagulation or electric energy for cutting to the instrument 205. Thus, since the third controller 300 can transmit the ON signal to the electrosurgical unit 230, electric energy can be supplied from the electrosurgical unit 230 to the end effector 204a and the end effector 205a without providing the switch elements to the instruments 204 and 205. Further, since the electrosurgical unit 230 is commonly provided for the instruments 201, 204 and 205, the configuration of the robotic surgical system 500c becomes simpler than when the electrosurgical units are individually provided for the instruments 201, 204 and 205.

Next, a case where the instrument 201 is attached to the robot arm 50 instead of the instrument 204 will be described. In the fourth embodiment, when the instrument 201 is attached to the robot arm 50, the first controller 310 acquires the tool information of the instrument 201 from the memory 201b. Then, the first controller 310 determines whether or not to assign the coagulation pedal 126R and the cutting pedal 125R to the instrument 201 based on the acquired tool information. If the instrument 201 is an electrosurgical instrument, the first controller 310 assigns the coagulation pedal 126R and the cutting pedal 125R to the instrument 201 mounted on the robotic arm 50 operated by the operating handle 110R. The third controller 300 is configured to transmit an ON signal to the switch element SW1a or the switch element SW1b while the coagulation pedal 126R or the cutting pedal 125R is operated. The switch element SW1a or the switch element SW1b is configured to turn on the coagulation supply line L1 or the cutting supply line L1 while receiving the ON signal from the third controller 300. Thus, the electrosurgical unit 230 supplies electrical energy for coagulation or cutting to the end effector 201a of the instrument 201. The other configuration of the fourth embodiment is the same as that of the third embodiment.

### (Fifth Embodiment)

The configuration of the robotic surgical system 500d according to a fifth embodiment will be described.

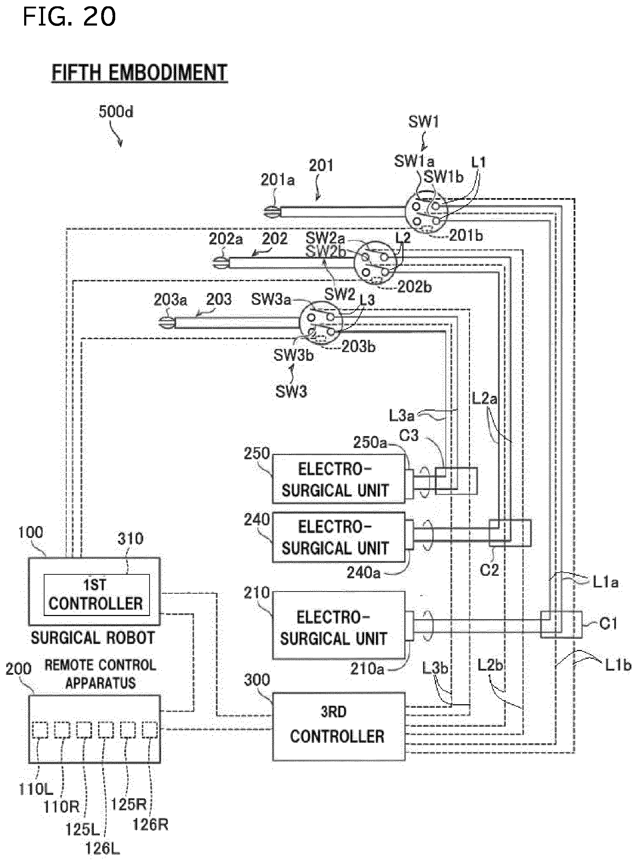

As shown in FIG. 20, the robotic surgical system 500d differs from the first or second embodiment in that the electrosurgical units are arranged separately for the three instruments. Specifically, an electrosurgical unit 210, an electrosurgical unit 240, and an electrosurgical unit 250 are disposed for the instruments 201, 202, and 203, respectively. The electrosurgical unit 210 includes a connector 210a, and an electric line L1a is connected to the connector 210a. The electrosurgical unit 240 includes a connector 240a, and an electric line L2a is connected to the connector 240a. The electrosurgical unit 250 includes a connector 250a, and an electric line L3a is connected to the connector 250a. The third controller 300 is not connected to the electrosurgical unit 210, the electrosurgical unit 240, and the electrosurgical unit 250, and does not transmit ON signals to the electrosurgical unit 210, the electrosurgical unit 240, and the electrosurgical unit 250. The electrosurgical unit 240 and the electrosurgical unit 250 are examples of "a second electrosurgical unit". The other configuration of the fifth embodiment is the same as that of the first embodiment.

### (Sixth Embodiment)

The configuration of the robotic surgical system 500e according to a sixth embodiment will be described.

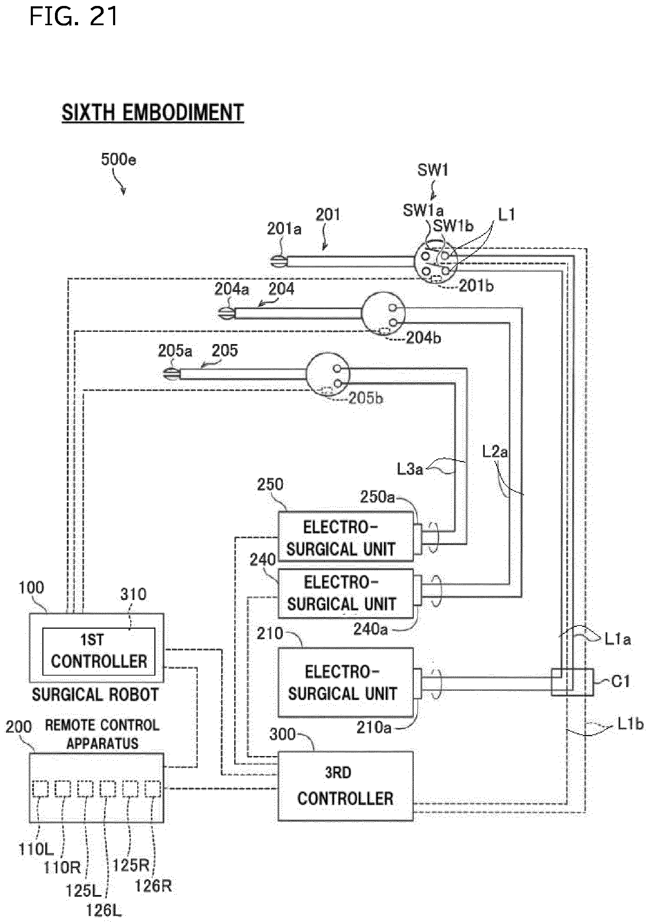

As shown in FIG. 21, the robotic surgical system 500e differs from the third or fourth embodiment in that the electrosurgical units are arranged separately for the three instruments. Specifically, an electrosurgical unit 210, an electrosurgical unit 240, and an electrosurgical unit 250 are disposed for the instruments 201, 204, and 205, respectively. The electrosurgical unit 210 includes a connector 210a, and an electric line L1a is connected to the connector 210a. The electrosurgical unit 240 includes a connector 240a, and an electric line L2a is connected to the connector 240a. The electrosurgical unit 250 includes a connector 250a, and an electric line L3a is connected to the connector 250a. The third controller 300 is not connected to the electrosurgical unit 210 and does not transmit an ON signal to the electrosurgical unit 210. On the other hand, the third controller 300 is connected to the electrosurgical unit 240 and the electrosurgical unit 250 and can transmit ON signals to the electrosurgical unit 240 and the electrosurgical unit 250, but the third controller 300 cannot receive device information of the electrosurgical unit 240 and the electrosurgical unit 250. The other configuration of the sixth embodiment is the same as that of the third embodiment.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

In the first to sixth embodiments described above, the first controller 310 and the third controller 300 are separate controllers, but the invention is not limited to this. For example, the first controller 310 and the third controller 300 may be configured as a single controller.

In the above first to sixth embodiments, the number of instruments (201, 202, 203) provided with switch elements among the three instruments is one or three, but the invention is not limited to this. For example, among the three instruments, two instruments may be provided with switch elements and one instrument may not be provided with a switch element.

In the above first to sixth embodiments, the electric line L1a (L2a, L3a) is provided with the relay connector C1 (C2, C3), but the invention is not limited to this. For example, the electric line L1a (L2a, L3a) does not have to be provided with the relay connector.

In the above first to sixth embodiments, three types of instruments (instruments 201, 202, and 203, or instruments 201, 204, and 205) are prepared for attachment to the robot arm 50, but the invention is not limited to this. For example, four or more types of instruments may be prepared for attachment to the robot arm 50. In this case, no switch element is provided for an instrument that is supplied electrical energy from an electrosurgical unit that receives an ON signal from the third control device 300. On the other hand, a switch element is provided for an instrument that is supplied electrical energy from an electrosurgical unit that does not receive an ON signal from the third control device 300.

In addition, in the above first to sixth embodiments, an example in which four robot arms 50 are provided has been shown, but the invention is not limited to this. In the invention, the number of robot arms 50 may be any other number as long as at least one or more robot arms 50 are provided.

In the first to sixth embodiments, the arm portion 51 and the positioner 30 are configured as a seven-axis articulated robot, but the invention is not limited to this. For example, the arm portion 51 and the positioner 30 may be configured as a articulated robot having an axis configuration other than a seven-axis articulated robot. An example of the axis configuration other than the seven-axis articulated robot is a six-axis or eight-axis articulated robot.

In the above first to fifth embodiments, the surgical robot 100 includes the medical cart 10, the positioner 30, and the arm base 40, but the invention is not limited to this, and for example, the positioner 30 and the arm base 40 are not necessarily required, and the surgical robot 100 may be composed of the medical cart 10 and one robot arm 50. In this case, four surgical robots 100 can be used.

## Claims

1. A robotic surgical system (500, 500a-500d) comprising:
a surgical instrument (201) that includes an end effector (201a), a supply line (L1) supplying an electric energy to the end effector and a switch element (SW1) turning on/off the supply line;
a robot arm (50d) to which the surgical instrument is detachably attached;
an electrosurgical unit (210, 230) that supplies the electric energy to the surgical instrument;
an input device (125R, 126R) that receives an input instructing to perform a surgical procedure using the surgical instrument; and
one or more control devices (300, 310) that transmit a signal to the switch element while the input received by the input device, wherein
the switch element turns on the supply line while the signal from the one or more control devices is received.

2. The robotic surgical system according to claim 1, wherein
the surgical instrument and the electrosurgical unit are connected by a first electric line (L1a), and the surgical instrument and the one or more control devices are connected by a second electric line (L1b).

3. The robotic surgical system according to claim 2, wherein
the first electric line includes a relay connector (C1) between the surgical instrument and the electrosurgical unit, and the second electric line connects the surgical instrument and the one or more control devices via the relay connector.

4. The robotic surgical system according to any one of claims 1 to 3, wherein
the one or more control devices do not transmit the signal to the switch element when the input is not received by the input device, and
the switching element does not turn on the supply line when the signal from the one or more control devices is not received.

5. The robotic surgical system according to any one of claims 1 to 4, further comprising:
a second surgical instrument (202) that includes a second end effector (202a), a second supply line (L2) that supplies a second electric energy to the second end effector and a second switch element (SW2) that turns on/off the second supply line;
a second robot arm (50a, 50b) to which the second surgical instrument is detachably attached;
a second electrosurgical unit (220, 240) that supplies the second electric energy to the second surgical instrument; and
a second input device (125L, 126L) that receives a second input instructing to perform a surgical procedure using the second surgical instrument, wherein
the one or more control devices transmit a second signal to the second switch element while the second input is received by the second input device, and
the second switch element turns on the second supply line while the second signal from the one or more control devices is received.

6. The robotic surgical system according to any one of claims 1 to 4, further comprising:
a second surgical instrument (202) that includes a second end effector (202a), a second supply line (L2) that supplies a second electric energy to the second end effector and a second switch element (SW2) that turns on/off the second supply line;
a second robot arm (50a, 50b) to which the second surgical instrument is detachably attached; and
a second input device (125L, 126L) that receives a second input instructing to perform a surgical procedure using the second surgical instrument, wherein
the electrosurgical unit is configured to supply the second electric energy to the second surgical instrument,
the one or more control devices transmit a second signal to the second switch element while the second input is received by the second input device, and
the second switch element turns on the second supply line while the second signal from the one or more control devices is received.

7. The robotic surgical system according to any one of claims 1 to 4, further comprising:
a second surgical instrument (204) including a second end effector (204a) and a second supply line that supplies a second electric energy to the second end effector;
a second robot arm (50a, 50b) to which the second surgical instrument is detachably attached;
a second electrosurgical unit (220, 240) that supplies the second electric energy to the second surgical instrument; and
a second input device (125L, 126L) that receives a second input instructing to perform a surgical procedure using the second surgical instrument, wherein
the one or more control devices transmit a second signal to the second electrosurgical unit while the second input is received by the second input device, and
the second electrosurgical unit supplies the second electric energy to the second surgical instrument while the second signal from the one or more control devices is received.

8. The robotic surgical system according to claim 7, wherein
when a third surgical instrument (205) including a third end effector (205a) and a third supply line that supplies a third electric energy to the third end effector is attached to the second robot arm in place of the second surgical instrument, the one or more control devices assign the second input device to the third surgical instrument, and the second input device receives a third input that instructs to perform a surgical procedure using the third surgical instrument,
the second electrosurgical unit is configured to supply the third electric energy to the third surgical instrument,
the one or more control devices transmit a third signal to the second electrosurgical unit while the third input is received by the second input device, and
the second electrosurgical unit supplies the third electric energy to the third surgical instrument while the third signal from the one or more control devices is received.

9. The robotic surgical system according to any one of claims 1 to 4, further comprising:
a second surgical instrument (204) including a second end effector (204a) and a second supply line that supplies a second electric energy to the second end effector;
a second robot arm (50a, 50b) to which the second surgical instrument is detachably attached; and
a second input device (125L, 126L) that receives a second input instructing to perform a surgical procedure using the second surgical instrument, wherein
the electrosurgical unit is configured to supply the second electric energy to the second surgical instrument,
the one or more control devices transmit a second signal to the second electrosurgical unit while the second input is received by the second input device, and
the electrosurgical unit supplies the second electric energy to the second surgical instrument while the second signal from the one or more control devices is received.

10. The robotic surgical system according to any one of claims 5 to 9, further comprising:
a remote operation apparatus (200) including a first operation device (110R) that operates the robot arm, a second operation device (110L) that operates the second robot arm, the input device and the second input device.

11. The robotic surgical system according to claim 10, wherein
the input device includes a first coagulation pedal (126R) that receives the input instructing to perform a coagulation as the surgical procedure using the surgical instrument and a first cutting pedal (125R) that receives the input instructing to perform a cutting as the surgical procedure using the surgical instrument and
the second input device includes a second coagulation pedal (126L) that receives the second input instructing to perform a coagulation as the surgical procedure using the second surgical instrument and a second cutting pedal (125L) that receives the second input instructing to perform a cutting as the surgical procedure using the second surgical instrument.

12. The robotic surgical system according to claim 10 or claim 11, wherein
the surgical instrument includes a first memory (201b) storing first tool information indicating an electrosurgical instrument,
the second surgical instrument includes a second memory (202b, 203b, 204b, 205b) storing second tool information indicating an electrosurgical instrument, and
the one or more control devices assign the input device to the surgical instrument when the first memory stores the first tool information, and assign the second input device to the second surgical instrument when the second memory stores the second tool information, and
the one or more control devices do not assign the input device to the surgical instrument when the first memory does not store the first information, and do not assign the second input device to the second surgical instrument when the second memory does not store the second information.

13. The robotic surgical system according to any one of claims 10 to 12, further comprising:
a third robot arm (50c) to which an endoscope is detachably attached; and
a display device (140, 610) configured to display an image (GR) captured by the endoscope, wherein
the one or more control devices control the display device to display a graphical user interface superimposed on the image, and the graphical user interface includes a first display area regarding to the surgical instrument attached to the robot arm and a second display area regarding to the second surgical instrument attached to the second robot arm.

14. The robotic surgical system according to claim 13, wherein
the one or more control devices control the display device so as to change and display the color of the first display area while the input is received by the input device, and so as to change and display the color of the second display area while the second input is received by the second input device.

15. The robotic surgical system according to claim 13 or claim 14, wherein
the remote operation apparatus includes a third input device (124), and
the one or more control devices control the third robot arm to which the endoscope is attached such that the third robot arm is operated by the first and second operation devices while the third input device is operated.
